# EUROPEAN PATENT SPECIFICATION

(11) **EP 1 759 731 B1**
(45) Date of publication and mention of the grant of the patent: **24.03.2010**
(21) Application number: 05425615.1
(22) Date of filing: 01.09.2005
(51) Int. Cl.: A61M 16/00

(54) **Ventilatory support device**
Vorrichtung zur Beatmungsunterstüzung
Appareil d'assistance respiratoire

(43) Date of publication of application: 07.03.2007
(73) Proprietor: Deas S.R.L., 48014 Castel Bolognese (IT)
(72) Inventor: Travan, Maurizio, 41037 Mirandola (IT)
(74) Representative: Ferreccio, Rinaldo

(56) References cited:
- EP-A- 1 078 646
- WO-A-98/48878
- WO-A-2004/009169
- DE-C- 471 385
- FR-A- 2 250 541
- US-A- 5 000 173

## Description

### Field of application

The present invention refers to a medical device for a patient's ventilatory support.

More in particular, the present invention refers to a ventilatory support device with administration of gas having a predetermined content of oxygen in continuously positive pressure, also called CPAP (continuous positive air pressure) assisted respiration device.

### Prior art

The continuous positive air pressure (CPAP) ventilatory support process represents a now well established method for the treatment of respiratory illnesses.

As is known, CPAP assisted respiration requires the patient's efficient spontaneous respiration and is used to treat illnesses of the hyaline membranes, wherein the alveoli have greater difficulty in expanding during inspiration and collapse more easily during expiration.

In particular, the loss of alveolar tone associated with other causes such as intraalveolar transudation, interstitial oedema and the reduction or lack of surface-active substance lead to a series of modifications of the respiratory mechanics and of the distribution of air in the lungs that result in serious and progressive respiratory insufficiency.

For this reason, to at least partially support the alveolar distension, a gas mixture, with a suitable oxygen content dosed in litres per minute (L/min) is administered to the patient that allows a pressure gradient to be obtained between the inside of the respiratory tree and the outside, active for the entire duration of the respiratory cycle.

The pressure gradient has the purpose of allowing a greater expansion of the alveoli during inspiration, preventing alveolar collapse at the end of expiration, keeping a determined positive end expiration pressure (PEEP), as well as reducing the respiration effort thus avoiding the occurrence of complications such as hypoxaemia, hypercapnia, metabolic and respiratory acidosis.

A known device commonly used for CPAP respiration comprises a main pipe extending along a given axis to connect an oxygen intake opening and a delivery opening suitable for being connected to a patient.

The intake opening is suitable for being connected to a pressurised gas source having a predetermined oxygen content.

Generally, to place the delivery opening in communication with the patient, a face mask or else a helmet is provided that are connected to the body of the device.

To ensure that a sufficient continuously positive pressure is maintained in the main pipe, the device is equipped with a valve, called PEEP valve, which allows a predetermined positive pressure to be generated in the entire system at the end of expiration.

Known mechanical or else hydraulic valves are used as PEEP valves.

In the prior art, normally to ensure ventilatory support it is necessary to ventilate the patient with a flow of gas of between about 60 and 120 litres per minute (L/min). The flow of gas at the delivery opening inspired on average by the patient is overall equal to 6-12 litres per minute.

The overall flow of gas pumped into the main duct is between about 60 and 120 litres per minute for the following reason.

During inspiration patient's instantaneous flows comprised between about 100 and 110 litres per minute occur.

The pumping of a high overall flow of gas does indeed ensure ventilatory support also when such instantaneous flows occur, and thus avoids the occurrence of undesired depressions in the system.

The known ventilatory support device, whilst allowing the desired positive pressure to be obtained during the entire respiration cycle, does nevertheless have some drawbacks that have not been overcome yet.

The main drawback lies in the that it is necessary to use hydraulic and mechanical valves that have some drawbacks linked to the wear and to the inertia of the mechanical parts and to the pollution by the water in the case of hydraulic valves, and they are also made up of a large number of components.

Another drawback is represented by the high consumption of gas (60-120 litres per minute compared to 6-12 litres per minute inspired on average by the patient).

As stated above, with the known device, it is indeed necessary to overdose the flow of gas to adapt the system to high instantaneous flows during inspiration.

Such drawback is particularly clear under emergency conditions or during transportation of the patient, i.e. when the gas is supplied only by a low-capacity cylinder.

The technical problem underlying the present invention is therefore that of making a ventilatory support device of the aforementioned type that allows the aforementioned drawbacks to be overcome, i.e. being a device that is simple to be manufactured and able to ensure a positive end expiration pressure without requiring the help of mechanical or hydraulic valves and with a reduced overall gas consumption.

Document US 5, 000, 173 shows the features of the preamble of Claim 1.

### Summary of the invention

The solution idea underlying the present invention is that of recovering a positive overall gas flow pressure in the main pipe and of exploiting the pressure of the recovered gas as end expiration valve.

On the basis of the present solution idea, the technical problem is solved by a ventilatory support device of the aforementioned type as defined in the appended claim 1.

Preferred embodiments of the device according to the invention are defined in the dependent claims.

Further characteristics and advantages of the invention shall become clearer from the following description of an embodiment thereof, given by way of indicative and non limiting example with reference to the attached figures.

### Brief description of the drawings

Figure 1 illustrates an axonometric view of a device according to the invention connected to two sources of oxygen and pressurised air.
Figure 2 illustrates an enlarged view of the device of figure 1.
Figure 3 illustrates a further enlarged view of the device according to the invention.
Figure 4a illustrates view from above of a front portion of the device illustrated in figure 3.
Figure 4b illustrates a section of the portion of figure 4a.
Figure 5 illustrates a section of the device of figure 3.
Figures 6a-6c illustrate sections of the device according to the invention wherein the gas flows during a respiration cycle are indicated.
Figure 7a illustrates an axonometric view of a rear portion of the device illustrated in figure 3.
Figure 7b illustrates a section of the rear portion illustrated in figure 7a.
Figure 8 illustrates a filter for the device according to the invention.
Figure 9 illustrates the same section of figure 5 wherein some relevant dimensions are indicated.

### Detailed description

With reference to the attached figures, a ventilatory support device in continuously positive air pressure (CPAP) according to the invention is indicated with reference number 10.

Preferably, the entire device 10 is of the disposable type and it is made entirely of plastic.

As it shall be better illustrated hereafter, in the illustrated solution, the device 10 is associated with a system 13 for feeding pressurised oxygen comprising a pure oxygen source 11 and an air source 12 having a known oxygen content.

Hereafter in the description, by way of example, reference will be made to the supply system, even if nothing forbids that the device 10 can be associated with a single pure oxygen source or with a single air source.

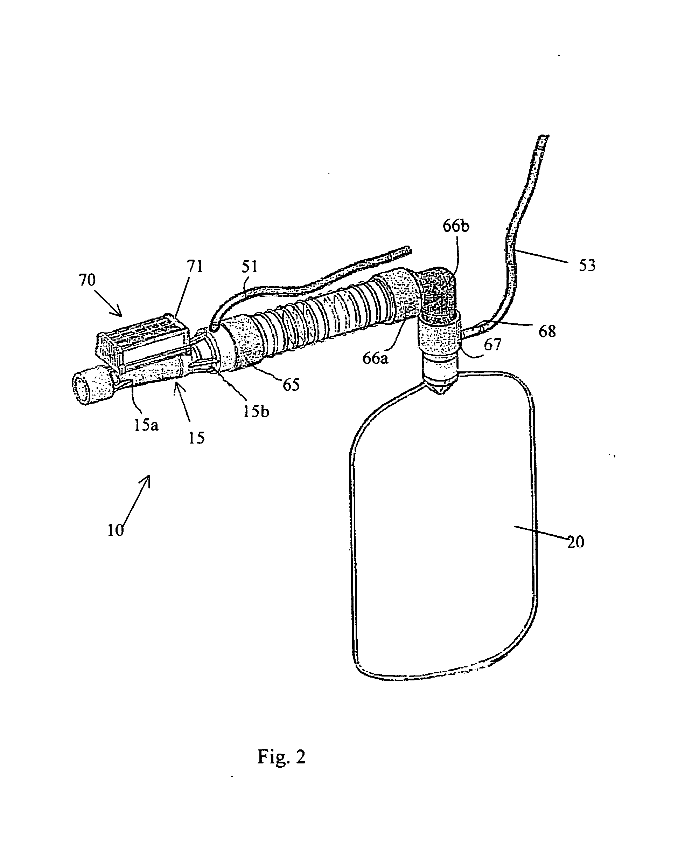

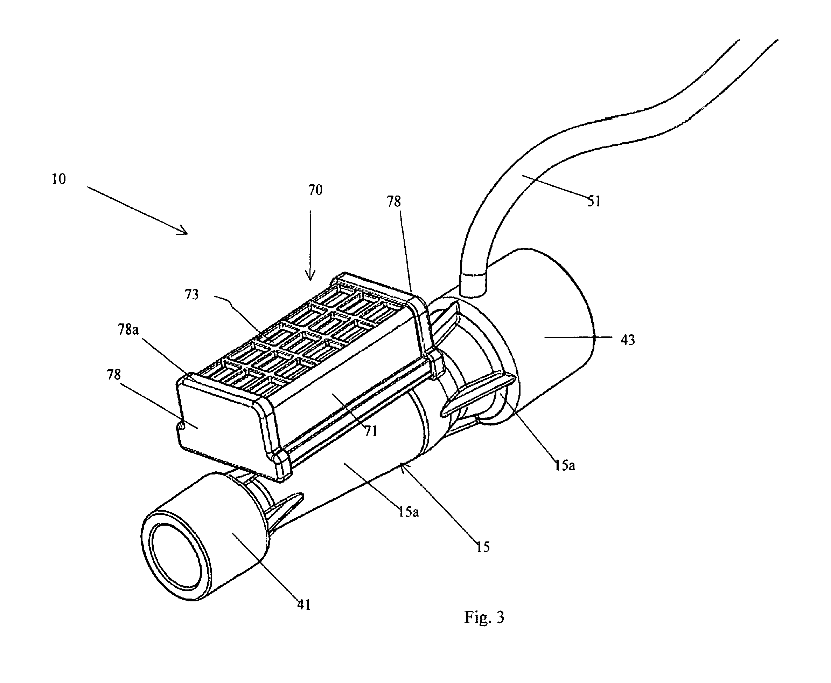

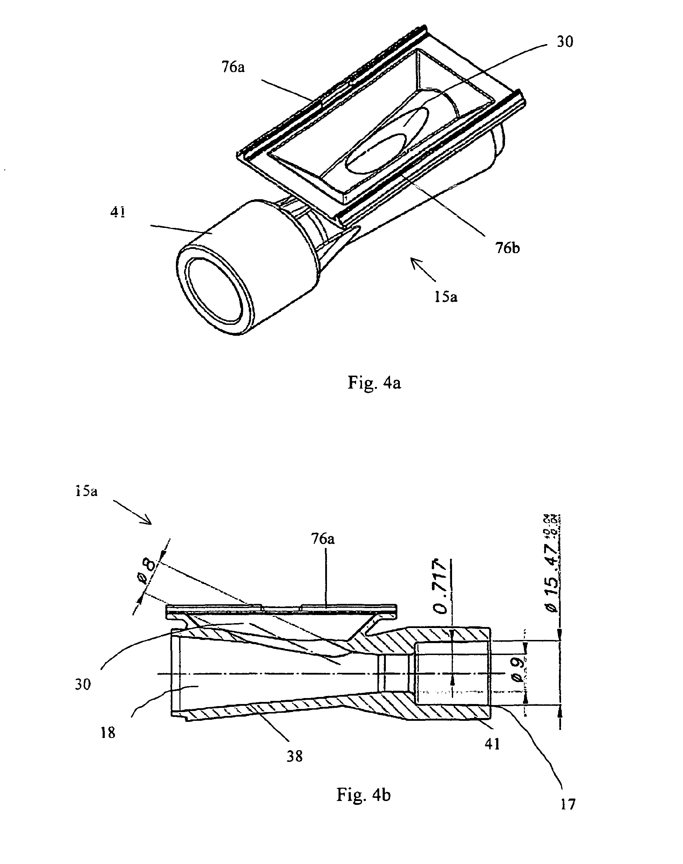

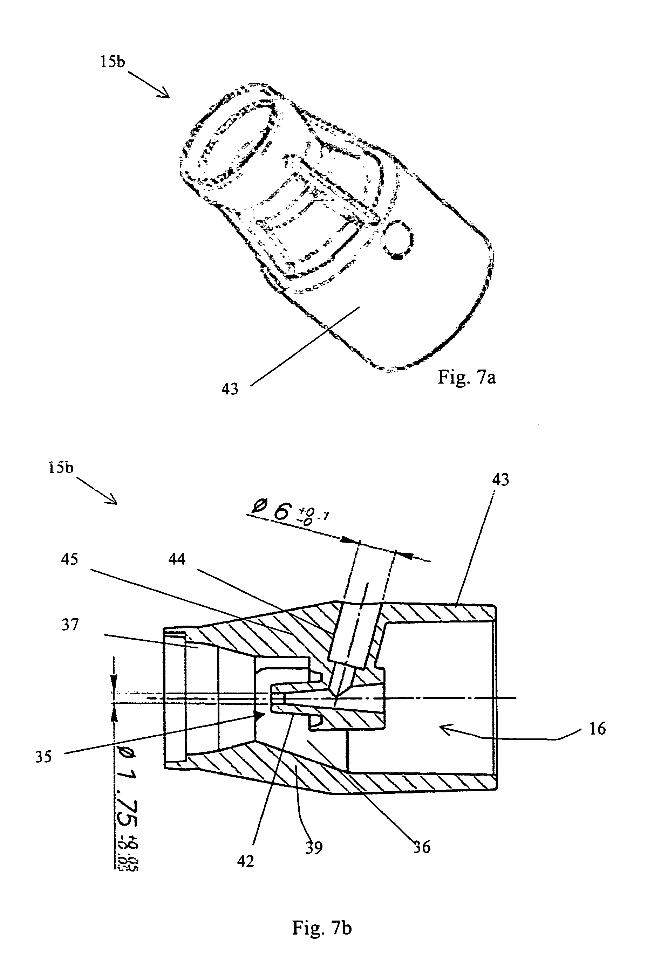

Even more specifically, with reference to figures 2-5 and 9, the device 10 comprises a substantially tubular shaped body 15 that is made, in the case of the illustrated solution, of a front portion 15a (figures 4a, 4b) and a rear portion 15b (figures 3, 7a, 7b) joined together.

In the body 15, the device 10 comprises a substantially rectilinear pipe 18 that extends along a determined axis X between an intake fitting, indicated with reference number 16 and formed in the rear portion 15b, and a delivery fitting 17 formed in the front portion 15a and that is suitable for being connected to a patient.

The patient can be connected directly to the delivery fitting 17, or else through a face mask not illustrated in the drawings that is fixed to the delivery fitting 17,

The delivery fitting 17 is substantially aligned along the axis X with the intake fitting 16.

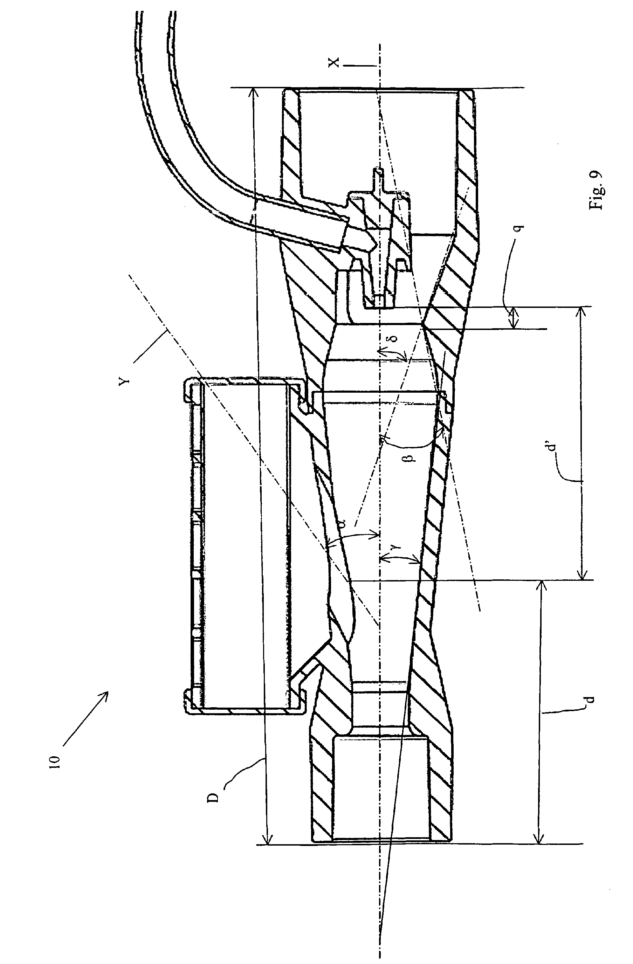

Preferably, the main pipe 18 is 123.5 mm long (length indicated with D in figure 9) and the intake fitting 16 and the delivery fitting 17 have a circular section with a diameter of 25 mm and about 15.5 mm respectively.

The device 10 comprises a substantially tubular injector 35 supported in the pipe 18 and orientated towards the delivery fitting 17. The injector 35 is substantially coaxial to the axis X of the main pipe 18 and it defines a substantially annular port 36 in the intake fitting 16 around the injector 35.

In practice, the device 10 comprises an injector 35, which is arranged along the axis X in the intake fitting 16 and defines an annular port 36 that coaxially surrounds the injector 35 itself.

Downstream of the injector 35, the main pipe 18 comprises a diverging inner portion 37, which diverges towards the delivery fitting 17.

The injector 35 is therefore upstream of the diverging portion 37 and substantially opposite the mouth of the delivery fitting 17, at a relatively short distance away from it. The distance is equal to about four times the diameter of the mouth of the delivery fitting 17, i.e. about 80 mm in the case of the illustrated solution.

The tubular injector 35 is connected to the pressurised oxygen sources 11 and 12, whereas the intake fitting 16 is placed in communication with an oxygen or oxygen and air reserve 20.

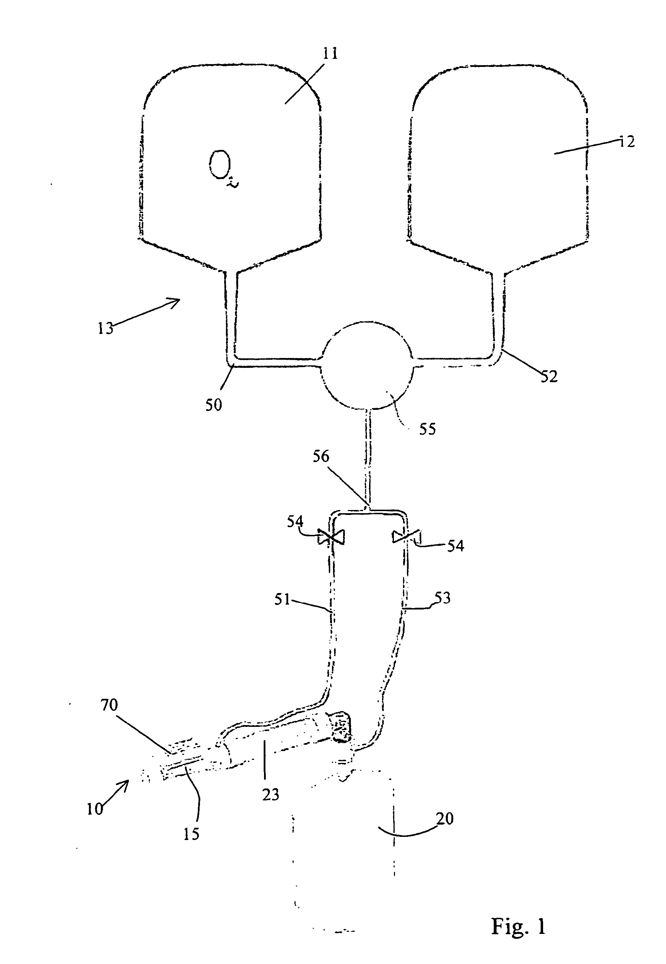

In the illustrated solution the gas reserve 20 is in the form of an elastic sack (figures 1 and 2) containing an air/oxygen mixture of suitable content that is connected, as it shall be explained more clearly hereafter, to the intake fitting 16 through a spiral-shaped tube 23.

The device 10 also comprises a discharge opening 30, which is open in the main pipe 18 in a zone between the injector 35 and the mouth of the delivery fitting 17 and which, as it shall be explained more clearly hereafter, discharges the gas expired by the patient in the expiration step.

In the case of the illustrated solution, the discharge opening 30 is placed at a predetermined distance d' away from the mouth of the injector 35 indicated in figure 9, substantially equal to the distance d from the mouth of the delivery fitting 17, wherein d is between 42 and 46 mm, and even more preferably it is equal to 43.95 mm.

Preferably, the discharge opening has an axis Y forming an angle α with the axis of the main pipe X.

The two axes X and Y converge towards the delivery fitting 17. The angle α is between about 22 and about 30 degrees, and even more preferably 26 degrees.

It should also be observed that the main pipe 18, starting from the intake fitting 16, has a Venturi portion, i,e. it comprises a narrowing 40 made of a first converging portion 39, which surrounds the injector 35 in the rear fitting 15b of the body 15, and the aforementioned diverging portion 37.

In the illustrated solution, the main pipe 18 also comprises a third converging portion 38 that extends for at least 1/2 , preferably for about 2/3, of the length of the main pipe 18 between the tubular injector 35 and the delivery fitting 17.

The discharge opening 30 is made in a substatially middle area of the third converging section 38, comprised between the tubular injector 35 and the mouth of the delivery fitting 17.

The diverging portion 37, which is made in the rear fitting 15b of the body 15, is connected directly to the third converging portion 38.

Preferably, the third converging portion 38 is longer than the first converging portion 39 and the diverging portion 37.

Preferably, the ratio between the length of the third converging portion 38, of the first converging portion 39 and of the diverging portion 37 is equal to about 4:1, 5:1.

In the case of the illustrated solution, the third converging portion 38 is about 48.3 mm long and forms a converging angle γ with the axis X of the main pipe 18, which is between about 4.500 and 7.000 degrees, and is preferably equal to 5.611 degrees.

The first converging portion 39 is about 14.5 mm long and forms a converging angle δ with the axis X of the main pipe 18 of between about 18 and 22 degrees, preferably 20 degrees.

It should be noted that the first converging portion 39 thus made allows the area of the mouth of the intake fitting 16 to be taken to such a value that the ratio between area of the outlet section of the injector 35 and the area of the minimum circular section of the converging portion 39 is between 1:50 and 1:150.

The diverging portion 37 is about 11.1 mm long and forms a diverging angle β with the axis X of the main pipe 18 of between about 13 and 17 degrees, preferably 15 degrees.

It should be also noted, as stated above, that only the first converging portion 39 of the Venturi portion surrounds the tubular injector 35. Basically, the tubular injector 35 is in withdrawn position with respect to the diverging portion 37 towards the mouth of the intake fitting 16.

Preferably, the distance q between the mouth of the injector 35 and the third diverging portion 37 is between 3 and 4 mm and this, as it shall be explained more clearly hereafter, allows a larger space to be obtained around the tubular injector 35 for the drawing of air.

It should also be noted that, in the illustrated solution, the pipe 18 comprises, near the mouth of the delivery fitting 17, an end portion 41 with constant section and, similarly, an end portion 43 with constant section near the intake fitting 16. These portions with constant section allow the device 10 to be respectively fitted to the spiral tube 23 and to a face machine, not illustrated in the drawings.

With reference to figure 7a, the injector 35 shall now be illustrated in detail.

The injector 35 comprises a frustum of cone portion 42 that is integral in a single body with the end portion 43 of the main pipe 18. The connection with the end portion 43 is made through a connection portion 45, which partially occupies the annular port 36.

In the portion 15b of the body 15 a passage 44 is also formed that is placed in fluid communication with the frustum of cone portion 42 on a side thereof to allow the intake of pressurised oxygen. The intake takes place through a flexible cannula 51 that is fixed to the passage 44.

As far as the oxygen feed is concerned, as stated above, the device 10 is connected to the oxygen/pressurised air feeding system 13 (figure 1), which is of the fixed type and usually arranged on a wall of a room and comprises the aforementioned sources 11 and 12.

The system 13 comprises a first line 50 and a second line 52 that connect the sources 11 and 12 to the device 10. Preferably, the device 10 is provided with a mixer 55, arranged downstream of the lines 50 and 52, intended to mix oxygen and air coming from the two sources 11 and 12, so as to obtain an air flow having a determined oxygen content.

The device 10 is also provided with a T-like joint, schematically indicated in figure 1 with reference number 56, which is arranged downstream of the mixer 55 and is suitable for dividing the flow of mixed gases respectively towards the injector 35 and the sack 20.

The T-like joint is connected to the injector 35 through the aforementioned cannula 51 and to the sack 20 through a cannula 53, The latter is connected to the sack 20 through a small tube 68.

The two cannulas 51 and 53 are fitted with flow regulators 54 for the regulation of the pressure/flow rate of the gas flow.

The connection of the sack 20 to the body 15 is completed by the aforementioned spiral tube 23 and by fittings 65, 66a, 66b and 67 (figure 2) of the known type.

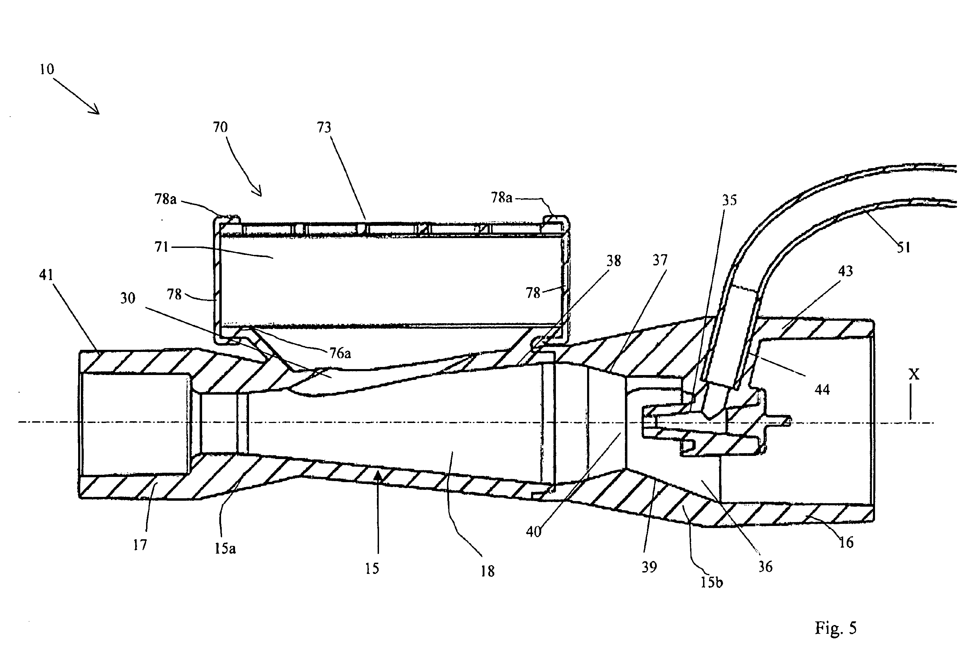

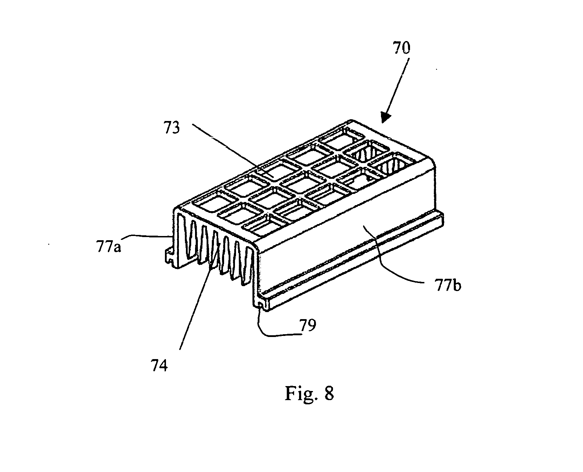

According to another characteristic of the present invention, the device 10 comprises a filter 70 (figures 5 and 8) fitted over the discharge opening 30 that is able to absorb the noise of the pressurised gas flows passing in the main pipe 18.

Preferably, the filter 70 comprises a box-shaped structure 71 (figure 8) fixed to the discharge opening 30 and provided with a grid 73, A pleated filtering body 74, preferably made from hydrophobic material capable of retaining the moisture contained in the gas expired by the patient, is fixed under the grid 73.

The pleated filter 74 also has an antibacterial function, i.e. it is able to protect the external environment from the possible emission of bacteria or microbes due to the patient's pathologies.

Preferably, the device 10 is provided with mutual engagement means to removably fix the filter 70 onto the discharge opening 30.

For such purpose the device 10 comprises opposite ribs 76a, 76b made integrally with the discharge opening 30 and that engage in corresponding grooves 79 formed on opposite sides 77a, 77b of the structure 71.

The filter 70 is completed by side covers 78 that laterally close the remaining sides of the structure 71 and engage through respective edges 78a at the ribs 76a, 76b and on the grid 73.

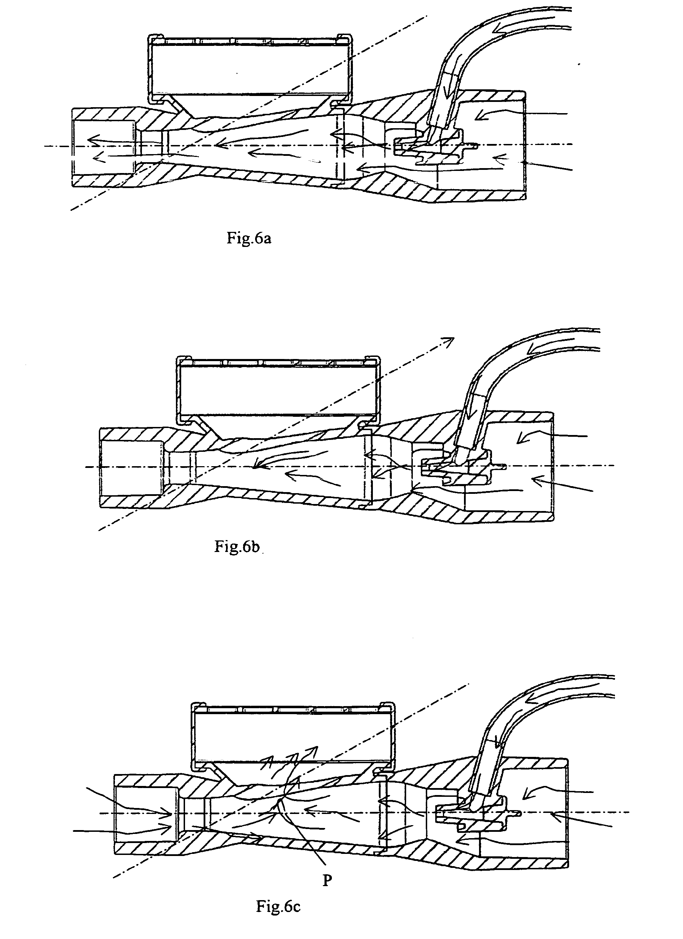

With reference to figures 1 and 6a-6c, the operation of the device 10 shall now be described.

Initially, once the device 10 has been connected to the air and oxygen sources 11 and 12, a step of setting the pressure of the gases is carried out by acting on the valves 54, so as to obtain a predetermined flow rate of the air with a known oxygen content to the injector 35 and to the sack 20.

The operating pressure range is preferably between 0.1 and 1 bar. The air is divided between the sack 20 and the injector 35 so that the flow rate of the air introduced into the sack 20 is equal to about 2-3 times that of the injector 35.

The flow rate of the jet coming out from the injector 35 varies between a minimum of 15 litres/minute and a maximum of 30 litres/minute, according to the patient's needs.

The jet coming out from the injector 35 is able to draw the air/oxygen gas mixture into the main pipe 18 from the sack 20 through the intake fitting, thus allowing a satisfactory amplification of the total gas flow to the delivery outlet 17 with respect to the flow coming out from the injector 35.

The overall flow is relatively low with respect to gas flows in the devices of the prior art and it is between about 35 and 60 litres/minute.

Downstream of the injector 35 a submerged jet consisting of a flow coming out from the injector 35 and whose current lines are not defined by any solid wall (at least in a region near the outlet) is obtained.

The main characteristic of this jet is the drawing action exerted thereby with respect to the surrounding fluid.

The amplification of the flow is also completed by the suction action of the gas through Venturi effect.

Basically, the amplification of the flow is given by the sum of two actions: a first action is the forcing and drawing of the gas in front of and surrounding the injector nozzle (submerged jet) and a second action is the suction of the gas situated behind the injector nozzle through Venturi effect.

Figure 6a illustrates the initial situation wherein the jet of air with known oxygen content is submerged in the gas flow drawn from the sack 20 through the intake fitting.

The drawing effect by the flow coming out from the injector 35 initially reduces the overall pressure of the flow in the diverging portion 37 of the main pipe 18, up to a negative value.

According to the invention, the flow downstream of the injector, i.e. in the zone of the main pipe 18 between the injector 35 and the delivery fitting, recovers the pressure that has been lost in the drawing step, thus allowing a satisfactory pressure value to be obtained in the main pipe 18.

An increase in the overall pressure of the flow of between about 2 and 16 cm of H₂O column is thus obtained in the main pipe 18.

It is to be noted that the positive pressure is completely recovered in a middle zone of the third converging portion 38 at the discharge opening 30.

It goes without saying that the greater the pressure value of the jet coming out from the injector 35 is, the greater the overall recovered pressure value is.

The device 10 is connected to a patient's respiratory apparatus for example to the mouth with the help of a face mask.

During inspiration, the patient creates a depression that, through the delivery fitting 17, propagates in the pipe 18, altering the ratio of the pressures generated therein by the jet.

This alteration involves an almost instant increase in the flow rate gain of the device that "following" the inspiration depression tries to keep the pressure positive, aim of the therapy.

It should be noted that, thanks to the recovery of pressure, the "following" of the inspiration depression and therefore the increase in flow occurs almost instantaneously.

In other words, with the device according to the invention, when, during inspiration, there are sudden changes of pressure equal to instantaneous flows of about 80-110 L per minute that alter the overall pressures at stake in the system, there is an almost instantaneous increase in the gas flow/pressure induced in the main pipe 18 through the opening 16 that satisfies the variations in pressure/instantaneous flows needed in the delivery fitting 17.

Basically, there is a modulation of the flow rate of the gas induced in the main pipe based upon the patient's instantaneous inspiration flows, The system thus dynamically adapts to the patient's inspiration.

This substantially instantaneous "following" of the inspiration depression is also promoted by the aforementioned arrangement of the injector 35 relatively close to the delivery fitting 17.

It should be noted that the aforementioned reduction of the overall gas flow in the main pipe of between about 35 and 60 litres per minute is strictly correlated to the dynamic adaptation of the system to the instantaneous flow variation generated by the patient.

In fact, in the device according to the invention, the overall gas flow in the main pipe momentarily increases only when required (increase in the induced flow rate) in response to a variation generated by the patient, otherwise the flow is kept at the same values of between 35 and 60 litres/minute which are sufficient to ensure the patient's ventilatory support. The latter, as stated above, respires about 6-12 litres of gas per minute.

Figure 6b illustrates the instant of switching from the inspiration step to the expiration step.

In the expiration step (figure 6c), the gas expired by the patient is placed in the main pipe 18 through the delivery fitting 17 in the opposite direction to the gas flow inlet. The expired gas counteracts the gas flow generated by the jet until a pressure equilibrium point is reached indicated with P in figure 6b.

The pressure equilibrium P is an unstable state of dynamic equilibrium and the two flows, in order to maintain such equilibrium, must come out through the discharge opening 30.

The deviation of the flows is also called flow switch.

Basically, the discharge opening 30 branches from the main pipe exactly where, in the expiration step, the overall recovery of pressure of the gas occurs and where the pressure equilibrium of the expired gas and of the inlet gas flow is reached, so that the two flows can deviate towards the discharge opening 30.

Figure 6c illustrates such condition wherein the two flows deviate to the outside through the filter 70.

At the end of the respiratory cycle, or rather during the pause that occurs between one respiratory cycle and the next, the pressure remains at a positive value (PEEP) corresponding to that reached in the state of dynamic equilibrium of the two flows (expiratory and generated by the jet).

It should be noted that thanks to the configuration of the illustrated device it is possible to regulate the inlet gas flow rate through the valves 54 within the aforementioned values, without altering the achievement of the pressure equilibrium at the discharge opening 30, and thus the positive end expiration pressure value, corresponding to 6-12 cm of H₂O column mentioned above.

The main advantage of the present invention is that of using the pressure recovery in the main pipe 18 to establish a state of dynamic equilibrium of the two flows in the main pipe, which constitutes an end expiration valve of the device.

The use of mechanical or hydraulic valves is thus avoided, thus obtaining a device having a simple structure with a small number of components.

A further advantage of the present invention lies in the possibility of regulating, according to the patient's needs, the pressure of the jet coming out from the injector 35 and therefore the overall flow rate of the flow of gas in the pipe within a wide range of values.

Thanks to the device according to the invention, by varying the pressure between a value of 0.2 and 1 bar, a satisfactory recovery of pressure is in any case ensured in the converging portion and therefore, in the expiration step, the desired pressure equilibrium that allows the deviation of the flows through the discharge opening is ensured.

In other words, it is possible to amplify the oxygen flow according to needs, without altering the pressures at stake in the device so as to ensure the establishment of the pressure equilibrium of the flows and their deviation into the discharge opening.

It should be observed that the desired pressure recovery is promoted by the substantially intermediate position of the discharge opening between the injector and the mouth of the delivery fitting.

A further advantage, with respect to devices of the prior art, is the low gas consumption (1/3 - 1/4 of total consumption).

In fact, as mentioned above, the device according to the invention allows a dynamic adaptation of the gas flow induced in the main pipe in response to pressure variations in the inspiration step, thus allowing less gas to be overall fed into the main pipe and without requiring an overdose.

In other words, the device according to the invention is able to maintain a pressure/flow rate equilibrium inside the system that adapts to the variations in pressure and flow rate that occur during a respiratory cycle both in the inspiration step (increase in gas flow in response to instantaneous high inspiration flows) and in the expiration step (pressure equilibrium at the discharge opening).

A further advantage of the present invention lies in the converging/diverging/converging configuration of the portions of the main pipe.

In fact, the diverging portion 37 promotes the entry of air, or gas mixture with known oxygen content, through the intake fitting and thus the amplification of the flow.

It should also be observed that the desired "gain" of the flow rate amplifier is promoted by the aforementioned sections ratio between the outlet mouth of the injector 35 and the smaller section of the first converging portion 39.

It should also be observed that the withdrawn position of the injector 35 with respect to the diverging portion 37 selected, as stated above, to have more space around the injector 35, further promotes the drawing of air.

Another advantage lies in the arrangement of the discharge opening having an axis that forms an angle converging with the axis of the main pipe. Such converging arrangement promotes the outlet of the flows in the expiration step.

A further advantage of the present invention lies in the fact that the injector and the intake fitting can be connected to the same gas source. This allows an amplification of the flow to be obtained without varying the gas composition.

A further advantage of the present invention lies in the presence of the filter mounted on the discharge opening that is able to absorb the noise of the pressurised gas flows as well as to retain the moisture contained in the gas expired by the patient.

A further advantage of the present invention lies in the possibility, when both the pressurised sources are not available, but only the oxygen source is (e.g. under all emergency conditions or during transportation when only the oxygen cylinder is normally present), to feed the jet with the available source and ensure the necessary high flows, thanks to the amplification in flow rate carried out by the device.

Of course, a man skilled in the art can bring numerous modifications and variants to the ventilatory support device described above, in order to satisfy contingent and specific requirements, all of which are covered by the scope of protection of the invention, as defined by the following claims.

## Claims

1. Ventilatory support device with administration of gas having a predetermined content of oxygen in continuously positive pressure, comprising a main pipe (18) extending between a gas intake fitting (16) provided with an intake opening and a gas delivery fitting (17) provided with a delivery mouth through which the gas is administered, an inner portion (37) of the pipe (18) diverging towards the delivery fitting (17), wherein the main pipe (18) comprises a Venturi portion made of a first converging portion (39) and of said diverging inner portion (37), said device comprising a substantially tubular injector (35) supported within the pipe (18) and orientated towards the delivery opening, and connected, when in use, to a pressurised gas source having a predetermined oxygen content (11, 12), a port (36) in communication with the intake opening, being defined around the injector (35), said device further comprising a discharge opening (30) for discharging expired gas, said opening being open in the main pipe (18) and said opening (30) being placed between the injector (35) and the delivery fitting (17) at a predetermined distance away from the injector (35), **characterised in that** said first converging portion (39) surrounds the tubular injector (35), and **in that** said discharge opening is positioned in a third portion (38) of the main pipe that extends converging towards the delivery fitting (17).

2. Device according to claim 1, **characterised in that** the tubular injector (35) comprises a frustum of cone portion (42) that is integral in a single body with an end portion (43) of the main pipe (18).

3. Device according to any one of the previous claims, **characterised in that** the discharge opening (30) is formed in a substantially middle zone between the tubular injector (35) and the delivery fitting (17).

4. Device according to claim 1, **characterised in that** it comprises regulation means (54) of the flow rate of the gas coming out from the injector (35) to obtain a positive pressure of the gas with a known oxygen content downstream of the injector (35).

5. Device according to claim 1, **characterised in that** the injector (35) is arranged substantially coaxial to the axis (X) of the main pipe (18).

6. Device according to claim 5, **characterised in that** the injector (35) is arranged substantially opposite the mouth of the delivery fitting (17) at a distance equal to about four times the diameter of the mouth of the delivery fitting (17).

7. Device according to claim 1, **characterised in that** the tubular injector (35) is in withdrawn position with respect to the diverging portion (37) towards the intake fitting (16).

8. Device according to any one of the previous claims, **characterised in that** the third portion (38) extends converging for at least half of the length of the main pipe (18) that is situated between the tubular injector (35) and the delivery fitting (17).

9. Device according to claim 8, **characterised in that** the ratio between the length of the third converging portion (38), of the first converging portion (39) and of the diverging portion (37) is about 4 : 1.5 : 1, respectively.

10. Device according to claim 1, **characterised in that** the third converging portion (38) forms a converging angle (γ) with the axis (X) of the main pipe (18), of between about 4.500 and 7.000 degrees.

11. Device according to claim 1, **characterised in that** the third converging portion (38) forms a converging angle (γ) with the axis (X) of the main pipe (18), of about 6 degrees.

12. Device according to claim 5, **characterised in that** the first converging portion (39) forms a converging angle (δ) with the axis (X) of the main pipe (18), of between about 18 and 22 degrees.

13. Device according to claim 9, **characterised in that** the diverging portion (37) forms a diverging angle (β) with the axis (X) of the main pipe (18), of between about 13 and 17 degrees.

14. Device according to any one of the previous claims, **characterised in that** the discharge opening (30) has an axis (Y) forming an angle (α) with the axis (X) of the main pipe (18), wherein said axes (X, Y) converge towards the delivery opening (17).

15. Device according to claim 14, **characterised in that** the angle between the axis (Y) of the discharge opening (30) and the axis (X) of the main pipe (18) is between about 22 and about 30 degrees.

16. Device according to any one of the previous claims, **characterised in that** it comprises a reserve sack (20) of gas with predetermined oxygen content that is connected to the intake opening (16).

17. Device according to any one of the previous claims, **characterised in that** it comprises means (50, 51, 52 53) for connecting the tubular injector (35) and the gas reserve (20) to the same pressurised gas source.

18. Device according to claim 17, **characterised in that** it comprises means (56) for fractioning the pressurised gas flow distributing it towards the injector (35) and the gas reserve (20) so as to maintain a concentration with known oxygen content.

19. Device according to any one of the previous claims, **characterised in that** it comprises a filter (70) fitted over the discharge opening (30) that is able to absorb the noise of the pressurised gas flows passing in the main pipe (18).

20. Device according to claim 19, **characterised in that** the filter (70) comprises a membrane made of hydrophobic material capable of retaining the moisture contained in the gas expired by the patient.

21. Device according to claim 20, **characterised in that** it comprises mutual engagement means for removably fixing the filter (70) to the discharge opening (30).

22. Device according to claim 20, **characterised in that** said mutual engagement means comprise opposite ribs (76a, 76b) made integrally with the discharge opening (30) and which engage in corresponding grooves (79) formed on opposite sides (77a, 77b) of the filter (70).

## Patentansprüche

1. Atemhilfevorrichtung mit Verabreichung von Gas mit einem vorbestimmten Sauerstoffgehalt unter ständigem Überdruck, die Folgendes umfasst: ein Hauptrohr (18), das zwischen einem mit einer Ansaugöffnung versehenen Gasansaugstutzen (16) und einem mit einer Zuführungsmündung, durch die das Gas verabreicht wird, versehenen Gaszuführstutzen (17) verläuft, wobei ein Innenabschnitt (37) des Rohrs (18) in Richtung auf den Zuführstutzen (17) divergiert, wobei das Hauptrohr (18) einen Venturi-Abschnitt aus einem ersten konvergierenden Abschnitt (39) und dem genannten divergierenden Innenabschnitt (37) umfasst, wobei die genannte Vorrichtung einen im Wesentlichen röhrenförmigen Injektor (35) umfasst, der in dem Rohr (18) gelagert und in Richtung der Zuführungsöffnung ausgerichtet und beim Gebrauch mit einer Druckgasquelle mit einem vorbestimmten Sauerstoffgehalt (11, 12) verbunden ist, wobei ein Anschluss (36) in Verbindung mit der Ansaugöffnung um den Injektor (35) herum definiert ist, wobei die genannte Vorrichtung ferner eine Abströmöffnung (30) zum Ablassen von ausgeatmetem Gas umfasst, wobei die genannte Öffnung im Hauptrohr (18) offen ist und die genannte Öffnung (30) zwischen dem Injektor (35) und dem Zuführstutzen (17) in einem vorbestimmten Abstand von dem Injektor (35) entfernt platziert ist, **dadurch gekennzeichnet, dass** der genannte erste konvergierende Abschnitt (39) den röhrenförmigen Injektor (35) umgibt, und **dadurch**, dass die genannte Abströmöffnung in einem dritten Abschnitt (38) des Hauptrohrs positioniert ist, der in Richtung auf den Zuführstutzen (17) konvergierend verläuft.

2. Vorrichtung nach Anspruch 1, **dadurch gekennzeichnet, dass** der röhrenförmige Injektor (35) einen kegelstumpfförmigen Abschnitt (42) umfasst, der einstückig in einem einzigen Körper mit einem Endabschnitt (43) des Hauptrohrs (18) vorgesehen ist.

3. Vorrichtung nach einem der vorherigen Ansprüche, **dadurch gekennzeichnet, dass** die Abströmöffnung (30) in einer im Wesentlichen mittleren Zone zwischen dem röhrenförmigen Injektor (35) und dem Zuführstutzen (17) ausgebildet ist.

4. Vorrichtung nach Anspruch 1, **dadurch gekennzeichnet, dass** sie ein Mittel (54) zum Regeln der Strömungsrate des aus dem Injektor (35) kommenden Gases umfasst, um einen Überdruck des Gases mit einem bekannten Sauerstoffgehalt stromabwärts von dem Injektor (35) zu erhalten.

5. Vorrichtung nach Anspruch 1, **dadurch gekennzeichnet, dass** der Injektor (35) im Wesentlichen koaxial zur Achse (X) des Hauptrohrs (18) angeordnet ist.

6. Vorrichtung nach Anspruch 5, **dadurch gekennzeichnet, dass** der Injektor (35) im Wesentlichen gegenüber der Mündung des Zuführstutzens (17) in einem Abstand von etwa dem Vierfachen des Durchmessers der Mündung des Zuführstutzens (17) angeordnet ist.

7. Vorrichtung nach Anspruch 1, **dadurch gekennzeichnet, dass** sich der röhrenförmige Injektor (35) in einer zurückgezogenen Position mit Bezug auf den divergierenden Abschnitt (37) in Richtung auf den Ansaugstutzen (16) befindet.

8. Vorrichtung nach einem der vorherigen Ansprüche, **dadurch gekennzeichnet, dass** der dritte Abschnitt (38) für wenigstens die Hälfte der Länge des Hauptrohrs (18) konvergierend verläuft, das sich zwischen dem röhrenförmigen Injektor (35) und dem Zuführstutzen (17) befindet.

9. Vorrichtung nach Anspruch 8, **dadurch gekennzeichnet, dass** das Verhältnis zwischen der Länge des dritten konvergierenden Abschnitts (38), des ersten konvergierenden Abschnitts (39) und des divergierenden Abschnitts (37) jeweils etwa 4 : 1,5 : 1 beträgt.

10. Vorrichtung nach Anspruch 1, **dadurch gekennzeichnet, dass** der dritte konvergierende Abschnitt (38) einen konvergierenden Winkel (γ) mit der Achse (X) des Hauptrohrs (18) zwischen etwa 4,500 und 7,000 Grad bildet.

11. Vorrichtung nach Anspruch 1, **dadurch gekennzeichnet, dass** der dritte konvergierende Abschnitt (38) einen konvergierenden Winkel (γ) mit der Achse (X) des Hauptrohrs (18) von etwa 6 Grad bildet.

12. Vorrichtung nach Anspruch 5, **dadurch gekennzeichnet, dass** der erste konvergierende Abschnitt (39) einen konvergierenden Winkel (δ) mit der Achse (X) des Hauptrohrs (18) zwischen etwa 18 und 22 Grad bildet.

13. Vorrichtung nach Anspruch 9, **dadurch gekennzeichnet, dass** der divergierende Abschnitt (37) einen divergierenden Winkel (β) mit der Achse (X) des Hauptrohrs (18) zwischen etwa 13 und 17 Grad bildet.

14. Vorrichtung nach einem der vorherigen Ansprüche, **dadurch gekennzeichnet, dass** die Abströmöffnung (30) eine Achse (Y) hat, die einen Winkel (α) mit der Achse (X) des Hauptrohs (18) bildet, wobei die genannten Achsen (X, Y) in Richtung auf die Zuführungsöffnung (17) konvergieren.

15. Vorrichtung nach Anspruch 14, **dadurch gekennzeichnet, dass** der Winkel zwischen der Achse (Y) der Abströmöffnung (30) und der Achse (X) des Hauptrohrs (18) zwischen etwa 22 und etwa 30 Grad beträgt.

16. Vorrichtung nach einem der vorherigen Ansprüche, **dadurch gekennzeichnet, dass** sie einen Reservebeutel (20) mit Gas mit einem vorbestimmten Sauerstoffgehalt umfasst, der mit der Ansaugöffnung (16) verbunden ist.

17. Vorrichtung nach einem der vorherigen Ansprüche, **dadurch gekennzeichnet, dass** sie Mittel (50, 51, 52, 53) zum Verbinden des röhrenförmigen Injektors (35) und der Gasreserve (20) mit derselben Druckgasquelle umfasst.

18. Vorrichtung nach Anspruch 17, **dadurch gekennzeichnet, dass** sie Mittel (56) zum Unterteilen des Druckgasflusses umfasst, um ihn in Richtung auf den Injektor (35) und die Gasreserve (20) zu verteilen, um eine Konzentration mit einem bekannten Sauerstoffgehalt beizubehalten.

19. Vorrichtung nach einem der vorherigen Ansprüche, **dadurch gekennzeichnet, dass** sie einen Filter (70) umfasst, der über der Abströmöffnung (30) montiert ist und das Rauschen des im Hauptrohr (18) fließenden Druckgases absorbieren kann.

20. Vorrichtung nach Anspruch 19, **dadurch gekennzeichnet, dass** der Filter (70) eine Membran aus einem hydrophoben Material umfasst, das die in dem vom Patienten ausgeatmeten Gas enthaltende Feuchtigkeit zurückhalten kann.

21. Vorrichtung nach Anspruch 20, **dadurch gekennzeichnet, dass** sie ein Ineinandergreifmittel zum entfernbaren Anbringen des Filters (70) an der Abströmöffnung (30) umfasst.

22. Vorrichtung nach Anspruch 20, **dadurch gekennzeichnet, dass** das genannte Ineinandergreifmittel gegenüberliegende Rippen (76a, 76b) umfasst, die in die Abströmöffnung (30) integriert sind und in entsprechende Nuten (79) eingreifen, die auf gegenüberliegenden Seiten (77a, 77b) des Filters (70) ausgebildet sind.

## Revendications

1. Dispositif de soutien pour respirateur avec une administration de gaz ayant une teneur en oxygène prédéterminée en pression positive en continu, comprenant un tuyau principal (18) s'étendant entre une installation d'arrivée du gaz (16) munie d'une ouverture d'arrivée et une installation de distribution du gaz (17) munie d'une ouverture de distribution par laquelle le gaz est administré, une partie interne (37) du tuyau (18) divergeant vers l'installation de distribution (17), où le tuyau principal (18) comprend une partie de Venturi faite d'une première partie convergente (39) et de la dite partie interne divergente (37), le dispositif comprenant un injecteur essentiellement tubulaire (35) soutenu dans le tuyau (18) et orienté vers l'ouverture de distribution, et connecté, pendant l'utilisation, à une source de gaz pressurisé ayant une teneur prédéterminée en oxygène (11, 12), un port (36) en communication avec l'ouverture d'arrivée étant défini autour de l'injecteur (35), le dit dispositif comprenant de plus une ouverture de déchargement (30) pour décharger le gaz expiré, la dite ouverture étant ouverte dans le tuyau principal (18) et la dite ouverture (30) étant placée entre l'injecteur (35) et l'installation de distribution (17) à une distance prédéterminée de l'injecteur (35), **caractérisé en ce que** la dite première partie convergente (39) entoure l'injecteur tubulaire (35), et **en ce que** la dite ouverture de déchargement est positionnée dans une troisième partie (38) du tuyau principal qui s'étend de manière convergente vers l'installation de distribution (17).

2. Dispositif selon la revendication 1, **caractérisé en ce que** l'injecteur tubulaire (35) comprend un frustrum de portion conique (42) qui est solidaire, en une pièce unique, d'une partie terminale (43) du tuyau principal (18).

3. Dispositif selon l'une quelconque des revendications précédentes, **caractérisé en ce que** l'ouverture de déchargement (30) est formée dans une zone essentiellement médiane entre l'injecteur tubulaire (35) et l'installation de distribution (17).

4. Dispositif selon la revendication 1, **caractérisé en ce qu'**il comprend un moyen de régulation (54) du taux de circulation du gaz sortant de l'injecteur (35) pour obtenir une pression positive du gaz avec une teneur en oxygène connue en aval de l'injecteur (35).

5. Dispositif selon la revendication 1, **caractérisé en ce que** l'injecteur (35) est disposé de manière essentiellement coaxiale à l'axe (X) du tuyau principal (18).

6. Dispositif selon la revendication 5, **caractérisé en ce que** l'injecteur (35) est disposé essentiellement en face de l'embouchure de l'installation de distribution (17) à une distance égale à environ quatre fois le diamètre de l'embouchure de l'installation de distribution (17).

7. Dispositif selon la revendication 1, **caractérisé en ce que** l'injecteur tubulaire (35) est en position en retrait par rapport à la partie divergente (37) vers l'installation d'arrivée (16).

8. Dispositif selon l'une quelconque des revendications précédentes, **caractérisé en ce que** la troisième partie (38) s'étend de manière convergente sur au moins la moitié de la longueur du tuyau principal (18) qui est situé entre l'injecteur tubulaire (35) et l'installation de distribution (17).

9. Dispositif selon la revendication 8, **caractérisé en ce que** le rapport entre la longueur de la troisième partie convergente (38), de la première partie convergente (39) et de la partie divergente (37) est d'environ 4/1,5/1, respectivement.

10. Dispositif selon la revendication 1, **caractérisé en ce que** la troisième partie convergente (38) forme un angle convergeant (γ) avec l'axe (X) du tuyau principal (18), compris entre environ 4,500 et 7,000 degrés.

11. Dispositif selon la revendication 1, **caractérisé en ce que** la troisième partie convergente (38) forme un angle convergent (γ)d'environ 6 degrés avec l'axe (X) du tuyau principal (18),.

12. Dispositif selon la revendication 5, **caractérisé en ce que** la première partie convergente (39) forme un angle convergent (δ) avec l'axe (X) du tuyau principal (18) compris entre environ 18 et 22 degrés.

13. Dispositif selon la revendication 9, **caractérisé en ce que** la partie divergente (37) forme un angle divergent (β) avec l'axe (X) du tuyau principal (18) compris entre environ 13 et 17 degrés.

14. Dispositif selon l'une quelconque des revendications précédentes, **caractérisé en ce que** l'ouverture de déchargement (30) a un axe (Y) formant un angle (α) avec l'axe (X) du tuyau principal (18), où les dits axes (X, Y) convergent vers l'ouverture de distribution (17).

15. Dispositif selon la revendication 14, **caractérisé en ce que** l'angle entre l'axe (Y) de l'ouverture de déchargement (30) et l'axe (X) du tuyau principal (18) est entre environ 22 et environ 30 degrés.

16. Dispositif selon l'une quelconque des revendications précédentes, **caractérisé en ce qu'**il comprend un sac de réserve (20) de gaz avec une teneur prédéterminée en oxygène qui est connecté à l'ouverture d'arrivée (16).

17. Dispositif selon l'une quelconque des revendications précédentes, **caractérisé en ce qu'**il comprend des moyens (50, 51, 52, 53) pour connecter l'injecteur tubulaire (35) et la réserve de gaz (20) à la même source de gaz pressurisé.

18. Dispositif selon la revendication 17, **caractérisé en ce qu'**il comprend un moyen (56) de fractionner le flux de gaz pressurisé en le distribuant vers l'injecteur (35) et la réserve de gaz (20) de façon à maintenir une concentration avec une teneur connue en oxygène.

19. Dispositif selon l'une quelconque des revendications précédentes, **caractérisé en ce qu'**il comprend un filtre (70) disposé sur l'ouverture de déchargement (30) qui est capable d'absorber le bruit du flux de gaz pressurisé passant dans le tuyau principal (18).

20. Dispositif selon la revendication 19, **caractérisé en ce que** le filtre (70) comprend une membrane faite de matériau hydrophobe capable de retenir l'humidité contenue dans le gaz expiré par le patient.

21. Dispositif selon la revendication 20, **caractérisé en ce qu'**il comprend un moyen d'engagement mutuel pour fixer le filtre (70) à l'ouverture de déchargement (30) de manière amovible.

22. Dispositif selon la revendication 20, **caractérisé en ce que** le dit moyen d'engagement mutuel comprend des côtes opposées (76a, 76b), formées solidaires de l'ouverture de déchargement (30) et qui s'engagent dans des gorges correspondantes (79) formées sur des côtés opposés (77a, 77b) du filtre (70).
